# EUROPEAN PATENT APPLICATION

(11) **EP 1 264 607 A1**
(43) Date of publication of application: **11.12.2002**
(21) Application number: 02253855.7
(22) Date of filing: 31.05.2002
(51) Int. Cl.: A61L 27/34, A61L 27/56, A61L 27/58

(54) **Attachment of absorbable tissue scaffolds to scaffold fixation devices**

(30) Priority: 05.06.2001 US 874218
(71) Applicant: ETHICON, INC., Somerville, NJ 08876 (US)
(72) Inventor: Contiliano, Joseph H., Stewartsville, NJ 08886 (US); Hammer, Joseph John, Bridgewater, NJ 08807 (US); Rezania, Alireza, Hillsborough, NJ 08844 (US); Scopelianos, Angelo G., Whitehouse Station, NJ 08889 (US); Vyakarnam, Murty Narayan, New York, NY 10025 (US); Zimmerman, Mark Charles, East Brunswick, NJ 08816 (US)
(74) Representative: Mercer, Christopher Paul

(57) **Abstract**

The present invention relates to tissue scaffold implant devices useful in the repair and/or regeneration of diseased and/or damaged musculoskeletal tissue and that include a foam tissue scaffold component fixedly attached to a scaffold fixation component via partial encapsulation of the fixation component by the foam scaffold component, and to methods of making such tissue scaffold implant device.

## Description

### FIELD OF THE INVENTION

The present invention relates to bioabsorbable tissue scaffold implant devices that facilitate repair or regeneration of diseased or damaged musculoskeletal tissue.

### BACKGROUND OF THE INVENTION

Tissue engineering (TE) is the application of engineering disciplines to either maintain existing tissue structures or to enable new tissue growth. This engineering approach generally includes the delivery of a biocompatible tissue scaffold that serves as an architectural support onto which cells may attach, proliferate, and synthesize new tissue to repair a wound or defect. Preferably, the tissue scaffolds should be made of bioabsorbable materials. Bioabsorbable tissue scaffolds are absorbed by the body after the body has synthesized new tissue to repair the wound or defect. Synthetic bioabsorbable biocompatible polymers are well known in the art and include aliphatic polyesters, homopolymers, and copolymers (random, block, segmented and graft) of monomers such as glycolic acid, glycolide, lactic acid, lactide (d, l, meso and mixtures thereof), ε-caprolactone, trimethylene carbonate and p-dioxanone.

Many absorbable tissue scaffolds have been recognized for use in the repair and regeneration of tissue. Porous mesh plugs composed of polyhydroxy acid polymers such as polylactide are used for healing bone voids. More recently, other tissue engineering scaffolds have been reported. These scaffolds are manufactured by a number of different processes, including the use of leachables to create porosity in the scaffold, vacuum foaming techniques and precipitated polymer gel masses. Polymer melts with fugitive compounds that sublimate at temperatures greater than room temperature are known. Textile-based, fibrous tissue scaffolds and biocompatible, bioabsorbable foam tissue scaffolds formed by lyophilization are known. A porous, open-cell foam of polyhydroxy acids with pore sizes from about 10 to about 200 µm is used for the in-growth of blood vessels and cells. The foam also could be reinforced with fibers, yarns, braids, knitted fabrics, scrims and the like.

Articular cartilage is a tissue that covers the articulating surfaces between bones in the joints and consists of two principal phases: a solid matrix and an interstitial fluid phase. The matrix, which gives cartilage its stiffness and strength, is produced and maintained by chondrocytes. The interstitial fluid phase provides viscoelastic behavior to the cartilage tissue. In repairing articular cartilage, the tissue engineering scaffold must be fastened to the underlying bone so as not to be displaced by the movement of the joint.

Methods of repairing articular cartilage are known. One known articular cartilage repair piece includes a backing layer of non-woven, felted fibrous material which is either uncoated or covered by a coating of tough, pliable material. Means for fastening the repair piece to the underlying bone include elongated fasteners, suturing, adhesive bonding, and mechanical interlocking in an undercut portion of bone.

One attachment method to hold a biomaterial in place until healing occurs includes several steps. First, sutures are anchored through the subchondral plate into bony tissue with at least two lines emerging from the surface. The two lines are then pulled through the implant and used to secure the cartilage repair materials in place.

To avoid the need for a multi-step process, several prior works describe devices that combine scaffolds and the means for fastening the scaffolds to the underlying bone. For example, in one known prosthetic, resorbable, articular cartilage scaffold, an absorbable base component is adapted for insertion into a pilot hole into cancellous bone, permitting anchoring of the device into that bone. The scaffold is fabricated of biocompatible, bioresorbable fibers. In forming the device, some of the fibers in the scaffold are compressively forced through holes in the top of the base component to attach the scaffold to the base. This compressive force, used to attach the scaffold to the base, may damage the scaffold.

In another known bioabsorbable cartilage repair system, a porous bioabsorbable insert is held in the side walls of a support frame by means of radially, outwardly-extending flanges that pass through windows in the side walls. Though this results in a single device combining a scaffold and a means for fastening the scaffolds to underlying bone, the scaffold must be manufactured to contain the radially, outwardly-extending flanges.

Biocompatible tissue scaffolds also have been prepared from biological-based polymers such as hyaluronic acid (HA), collagen, alginates, chitosan and blends thereof. Three-dimensional porous foams and nonwoven structures of various biopolymers such as HA and collagen are known.

There are a number of tissue engineered scaffold devices that serve as architectural supports for the growth of new tissue structures. Although means for fastening these devices to the underlying bone have been described, the limits on the previously disclosed methods and devices include the need for a multistep fastening process, possible damage to the scaffold, and scaffolds that must be manufactured in very specific shapes to attach to the fastening means. Accordingly, there is a need for tissue engineering scaffold devices to be firmly affixed to hard tissue, such as bone or cartilage, wherein the scaffolds are held in place in the fixation device while tissue ingrowth occurs.

### SUMMARY OF THE INVENTION

The present invention relates to tissue scaffold implant devices comprising a scaffold fixation component and a foam tissue scaffold component fixedly attached to the scaffold fixation component and to methods of making such tissue scaffold implant devices. The foam scaffold component comprises an open-cell pore structure effective to facilitate tissue infiltration and growth into the foam tissue scaffold, and the foam scaffold component is affixed to the scaffold fixation component via partial encapsulation of the fixation component by the foam scaffold component. The implant device preferably is made by placing the fixation component within a mold of selected configuration, in a selected position and orientation, adding to the mold a solution of a selected polymeric material in a suitable solvent therefore, separating the polymer solution in the mold into a solvent phase and a polymer phase, and removing the solvent phase from the mold, thereby providing a foam tissue scaffold component partially encapsulating the fixation component, thus providing partial encapsulation of the fixation component by the polymeric foam component, thereby providing fixed attachment of the foam scaffold component to the fixation component with no additional means of attachment. Such devices are useful in the repair and/or regeneration of diseased and/or damaged musculoskeletal tissue and are an improvement over devices requiring multistep fixation processes.

### BRIEF DESCRIPTION OF THE FIGURES

Figure 1 is a top perspective view of a device of the present invention.
Figure 2 is a bottom perspective view of a device of the present invention.
Figure 3 is a cross-sectional view of a device of the present invention.
Figure 3(a) is a cross-sectional view of a device of the present invention.
Figure 4 is a scanning electron micrograph of a cross-section of a device according to the present invention.
Figure 5 is a scanning electron micrograph of a magnified portion of the device depicted in Figure 4.
Figure 6 is a cross-sectional view of one embodiment of a mold set-up useful with the present invention.
Figure 7 is a perspective view of an alternate embodiment of a mold set-up useful with the present invention.
Figure 8 is a sectional view of a portion of the mold set-up of Figure 7.
Figure 9 is a photographic image of a device according to the present invention that was cultured with cells.
Figure 10a is a histological section of a portion of a device according to the present invention seeded with cells.
Figure 10b is a magnified portion of Figure 10a, showing a histological section of a portion of a device according to the present invention seeded with cells.
Figure 11 is a histological section of a portion of a device according to the present invention following implantation in a trochlear groove site.
Figure 12 is a scanning electron micrograph of a cross-section of a device according to the present invention using a biopolymer to form the scaffold.
Figure 13 is a scanning electron micrograph of a cross-section of a device according to the present invention comprising a reinforcing component.

### DETAILED DESCRIPTION OF THE INVENTION

In the repair of articular cartilage, the structure of the implant must be effective to facilitate tissue ingrowth. A preferred tissue ingrowth-promoting structure is one where the cells of the foam scaffold component are open and of sufficient size to permit cell growth therein. An effective pore size is one in which the pores have an average diameter in the range of from about 100 to about 1,000 microns, more preferably, from about 150 to about 500 microns.

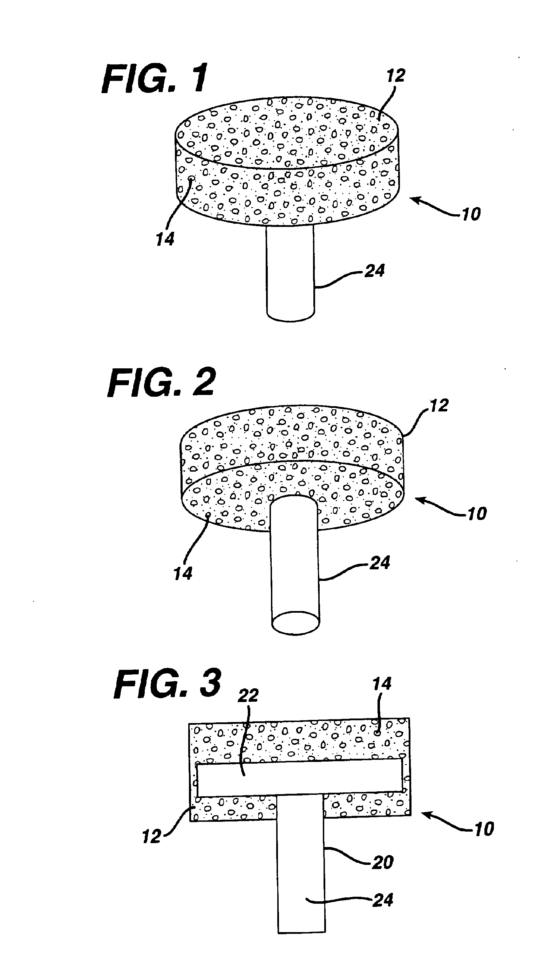

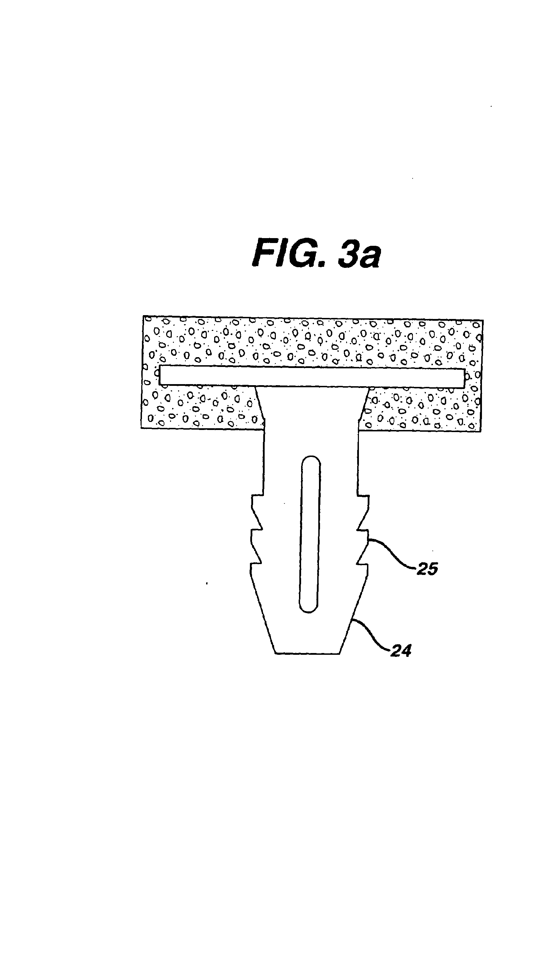

Referring to Figures 1 through 3, implant 10 includes polymeric foam scaffold component 12 and fixation component 20. Foam component 12 preferably contains pores 14 having an open-cell pore structure. Fixation component 20 includes scaffold support 22 and anchoring post 24. A preferred fixation component for use in the present invention is shown in Figure 3a. As shown therein, anchoring post 24 may contain ribs, serrations, or other surface roughness or engagement means 25 that improve attachment of anchoring post 24 to the implant site.

Implant 10 must have sufficient structural integrity and physical properties to facilitate ease of handling in an operating room environment. Polymeric foam scaffold component 12 and fixation component 20 must be fixedly attached so as to not separate before, during or after the surgical procedure. Sufficient strength and physical properties are developed in the implant through the selection of materials used to form the foam scaffold and fixation components, and the manufacturing process. In the embodiment as shown in Figure 3, foam component 12 substantially encapsulates scaffold support 22 of fixation component 20. This encapsulation serves as the means of fixedly attaching foam component 12 to fixation component 20 without the need for additional attachment means.

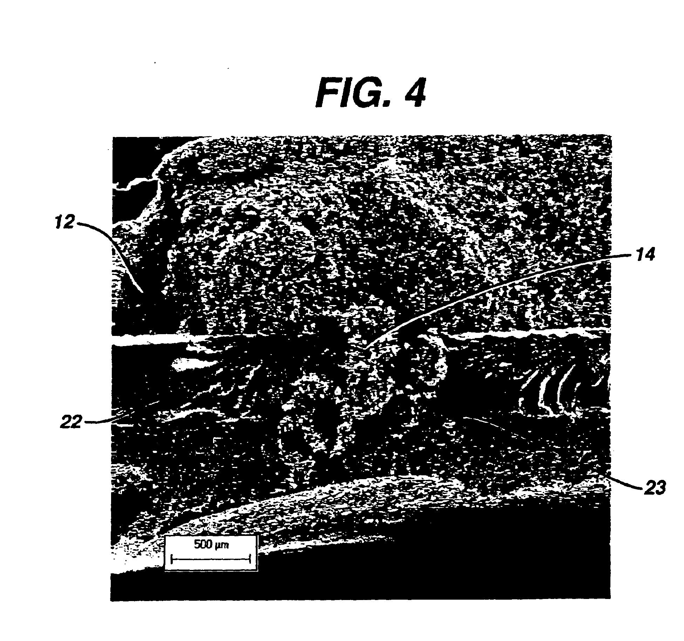

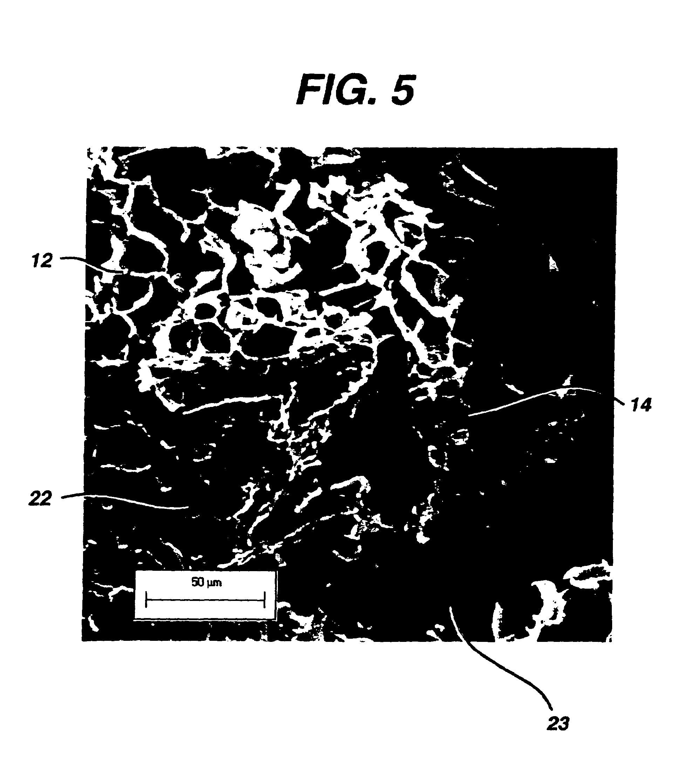

In a preferred embodiment shown in Figures 4 and 5, scaffold support 22 of fixation component 20 has through-holes 23 such that foam scaffold component 12 is integrated with scaffold support 22 such that pores 14 of foam scaffold component 12 penetrate through-holes 23 and interlock with scaffold support 22.

In a preferred embodiment, the fixation component and the foam scaffold component preferably comprise bioabsorbable polymers. Such a device utilizing the method of attachment of the present invention will result in a tissue-engineered scaffold implant device that is fully absorbable by the body.

A variety of bioabsorbable polymers can be used to make tissue-engineered scaffold implant devices according to the present invention. Examples of suitable biocompatible, bioabsorbable polymers include polymers selected from the group consisting of aliphatic polyesters, poly(amino acids), copoly(ether-esters), polyalkylene oxalates, polyamides, tyrosine-derived polycarbonates, poly(iminocarbonates), polyorthoesters, polyoxaesters, polyamidoesters, polyoxaesters containing amine groups, poly(anhydrides), polyphosphazenes, biomolecules (i.e., biopolymers such as collagen, elastin, bioabsorbable starches, etc.) and blends thereof. For the purpose of this invention aliphatic polyesters include, but are not limited to, homopolymers and copolymers of lactide (which includes lactic acid, D-, L- and meso lactide), glycolide (including glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one (including its dimer 1,5,8,12-tetraoxacyclotetradecane-7,14-dione), 1,5-dioxepan-2-one, 6,6-dimethyl-1,4-dioxan-2-one, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one and polymer blends thereof. Poly(iminocarbonates), for the purpose of this invention, are understood to include those polymers as described by Kemnitzer and Kohn, in the Handbook of Biodegradable Polymers, edited by Domb, et. al., Hardwood Academic Press, pp. 251-272 (1997). Copoly(ether-esters), for the purpose of this invention, are understood to include those copolyester-ethers as described in the Journal of Biomaterials Research, Vol. 22, pages 993-1009, 1988 by Cohn and Younes, and in Polymer Preprints (ACS Division of Polymer Chemistry), Vol. 30(1), page 498, 1989 by Cohn (e.g. PEO/PLA). Polyalkylene oxalates, for the purpose of this invention, include those described in U.S. Patent Numbers 4,208,511; 4,141,087; 4,130,639; 4,140,678; 4,105,034; and 4,205,399. Polyphosphazenes, co-, ter- and higher order mixed monomer based polymers made from L-lactide, D,L-lactide, lactic acid, glycolide, glycolic acid, para-dioxanone, trimethylene carbonate and ε-caprolactone are described by Allcock in The Encyclopedia of Polymer Science, Vol. 13, pages 31-41, Wiley Intersciences, John Wiley & Sons, 1988 and by Vandorpe, et al in the Handbook of Biodegradable Polymers, edited by Domb, et al., Hardwood Academic Press, pp. 161-182 (1997). Polyanhydrides include those derived from diacids of the form HOOC-C₆H₄-O-(CH₂)ₘ-O-C₆H₄-COOH, where m is an integer in the range of from 2 to 8, and copolymers thereof with aliphatic alpha-omega diacids of up to 12 carbons. Polyoxaesters, polyoxaamides and polyoxaesters containing amines and/or amido groups are described in one or more of the following U.S. Patent Nos. 5,464,929; 5,595,751; 5,597,579; 5,607,687; 5,618,552; 5,620,698; 5,645,850; 5,648,088; 5,698,213; 5,700,583; and 5,859,150. Polyorthoesters include those described by Heller in Handbook of Biodegradable Polymers, edited by Domb, et al, Hardwood Academic Press, pp. 99-118 (1997).

The fixation component of the invention may be composed of non-absorbable materials, such as biocompatible metals, including but not limited to stainless steel, cobalt chrome, titanium and titanium alloys; or bio-inert ceramic particles, including but not limited to alumina, zirconia, and calcium sulfate particles; or particles of non-bioabsorbable polymers, including but not limited to polyethylene, polyvinyl alcohol (PVA), polymethylmethacrylte (PMMA), silicone, polyethylene oxide (PEO), polyethylene glycol (PEG), and polyurethanes; or biocompatible and resorbable biopolymers. As used herein, the term "biopolymer" is understood to encompass naturally occurring polymers, as well as synthetic modifications or derivatives thereof. Such biopolymers include, without limitation, hyaluronic acid, collagen, recombinant collagen, cellulose, elastin, alginates, chondroitin sulfate, chitosan, chitin, keratin, silk, and blends thereof. These biopolymer scaffolds can be further modified to enhance their mechanical or degradation properties by introducing cross-linking agents or changing the hydrophobicity of the side residues.

As used herein, the term "glycolide" is understood to include polyglycolic acid. Further, the term "lactide" is understood to include L-lactide, D-lactide, blends thereof, and lactic acid polymers and copolymers.

Currently, aliphatic polyesters are among the preferred absorbable polymers for use in making the foam scaffold component according to the present invention. Aliphatic polyesters can be homopolymers copolymers (random, block, segmented, tapered blocks, graft, triblock, etc.) having a linear, branched or star structure. Suitable monomers for making aliphatic homopolymers and copolymers may be selected from the group consisting of, but are not limited to, lactic acid, lactide (including L-, D-, meso and D,L mixtures), glycolic acid, glycolide, ε-caprolactone, p-dioxanone (1,4-dioxan-2-one), trimethylene carbonate (1,3-dioxan-2-one), δ-valerolactone, β-butyrolactone, ε-decalactone, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, γ-butyrolactone, 1,4-dioxepan-2-one, 1,5-dioxepan-2-one, 6,6-dimethyl-dioxepan-2-one and 6,8-dioxabicycloctane-7-one.

Elastomeric copolymers also are particularly useful in the present invention. Suitable elastomeric polymers include those with an inherent viscosity in the range of about 1.2 dL/g to about 4 dL/g, more preferably about 1.2 dL/g to about 2 dL/g and most preferably about 1.4 dL/g to about 2 dL/g, as determined at 25°C in a 0.1 gram per deciliter (g/dL) solution of polymer in hexafluoroisopropanol (HFIP). Further, suitable elastomers exhibit a high percent elongation and a low modulus, while possessing good tensile strength and good recovery characteristics. In the preferred embodiments of this invention, the elastomer from which the polymer foam scaffold component is formed exhibits a percent elongation (e.g., greater than about 200 percent and preferably greater than about 500 percent). In addition to these elongation and modulus properties, suitable elastomers also should have a tensile strength greater than about 500 psi, preferably greater than about 1,000 psi, and a tear strength of greater than about 50 lbs/inch, preferably greater than about 80 lbs/inch.

Exemplary bioabsorbable, biocompatible elastomers include but are not limited to elastomeric copolymers of ε-caprolactone and glycolide with a mole ratio of ε-caprolactone to glycolide of from about 35:65 to about 65:35, more preferably from 45:55 to 35:65; elastomeric copolymers of ε-caprolactone and lactide where the mole ratio of ε-caprolactone to lactide is from about 35:65 to about 65:35 and more preferably from 45:55 to 30:70 or from about 95:5 to about 85:15; elastomeric copolymers of p-dioxanone (1,4-dioxan-2-one) and lactide where the mole ratio of p-dioxanone to lactide is from about 40:60 to about 60:40; elastomeric copolymers of ε-caprolactone and p-dioxanone where the mole ratio of ε-caprolactone to p-dioxanone is from about from 30:70 to about 70:30; elastomeric copolymers of p-dioxanone and trimethylene carbonate where the mole ratio of p-dioxanone to trimethylene carbonate is from about 30:70 to about 70:30; elastomeric copolymers of trimethylene carbonate and glycolide where the mole ratio of trimethylene carbonate to glycolide is from about 30:70 to about 70:30; elastomeric copolymers of trimethylene carbonate and lactide where the mole ratio of trimethylene carbonate to lactide is from about 30:70 to about 70:30, or blends thereof. Examples of suitable bioabsorbable elastomers are described in U.S. Patent Nos. 4,045,418, 4,057,537 and 5,468,253.

One of ordinary skill in the art will appreciate that the selection of a suitable polymer or copolymer for forming the foam scaffold component depends on several factors. The more relevant factors in the selection of the appropriate polymer(s) that is used to form the foam scaffold component include bioabsorption (or bio-degradation) kinetics; *in vivo* mechanical performance; cell response to the material in terms of cell attachment, proliferation, migration and differentiation; and biocompatibility. Other relevant factors which, to some extent, dictate the *in vitro* and *in vivo* behavior of the polymer include the chemical composition, spatial distribution of the constituents, the molecular weight of the polymer and the degree of crystallinity.

The ability of the material substrate to resorb in a timely fashion in the body environment is critical. But the differences in the absorption time under *in vivo* conditions also can be the basis for combining two different copolymers. For example, a copolymer of 35:65 ε-caprolactone and glycolide (a relatively fast absorbing polymer) is blended with 40:60 ε-caprolactone and L-lactide copolymer (a relatively slow absorbing polymer) to form the foam scaffold component. Depending upon the processing technique used, the two constituents can be either randomly inter-connected bicontinuous phases, or the constituents could have a gradient-like architecture in the form of a laminate type composite with a well integrated interface between the two constituent layers. The microstructure of these foams can be optimized to regenerate or repair the desired anatomical features of the tissue that is being engineered.

In one embodiment it is desirable to use polymer blends to form structures which transition from one composition to another composition in a gradient-like architecture. Foams having this gradient-like architecture are particularly advantageous in tissue engineering applications to repair or regenerate the structure of naturally occurring tissue such as cartilage (articular, meniscal, septal, tracheal, etc.). For example, by blending an elastomer of ε-caprolactone-co-glycolide with ε-caprolactone-co-lactide (e.g., with a mole ratio of about 5:95) a foam may be formed that transitions from a softer spongy material to a stiffer more rigid material in a manner similar to the transition from cartilage to bone. Clearly, one of ordinary skill in the art will appreciate that other polymer blends may be used for similar gradient effects, or to provide different gradients (e.g., different absorption profiles, stress response profiles, or different degrees of elasticity.

In another embodiment of the present invention, the polymeric foam scaffold component may contain reinforcing elements. The reinforcing elements of the tissue engineered scaffold implant device of the present invention can be comprised of any absorbable or non-absorbable biocompatible material, including textiles with woven, knitted, warped knitted (i.e., lace-like), non-woven and braided structures. In an exemplary embodiment the reinforcing component has a mesh-like structure. In any of the above structures, mechanical properties of the material can be altered by changing the density or texture of the material, or by embedding particles in the material. The fibers used to make the reinforcing component can be monofilaments, yarns, threads, braids or bundles of fibers. These fibers can be made of any biocompatible material, including bioabsorbable materials such as polylactic acid (PLA), polyglycolic acid (PGA), polycaprolactone (PCL), polydioxanone (PDO), trimethylene carbonate (TMC), polyvinyl alcohol (PVA), copolymers or blends thereof. In one embodiment, the fibers are formed of a polylactic acid and polyglycolic acid copolymer at a 95:5 mole ratio.

In another embodiment, the fibers that form the reinforcing material can be made of a bioabsorbable glass. Bioglass, a silicate containing calcium phosphate glass, or calcium phosphate glass with varying amounts of iron particles added to control resorption time, are examples of materials that could be spun into glass fibers and used for the reinforcing material.

The reinforcing material also may be formed from a thin, perforation-containing elastomeric sheet with perforations to allow tissue ingrowth. Such a sheet could be made of blends or copolymers of polylactic acid (PLA), polyglycolic acid (PGA) and polycaprolactone (PCL).

In one embodiment, filaments that form the reinforcing material may be coextruded to produce a filament with a sheath/core construction. Such filaments are comprised of a sheath of biodegradable polymer that surrounds one or more cores comprised of another biodegradable polymer. Filaments with a fast-absorbing sheath surrounding a slower-absorbing core may be desirable in instances where extended support is necessary for tissue ingrowth.

The polymeric foam scaffold component of the tissue-engineered scaffold implant device may be formed as a foam by a variety of techniques well known to those having ordinary skill in the art. For example, the polymeric starting materials may be foamed by lyophilization, supercritical solvent foaming (i.e., as described in EP 464,163), gas injection extrusion, gas injection molding or casting with an extractable material (e.g., salts, sugar or similar suitable materials).

In one embodiment, the foam scaffold component of the tissue-engineered implant devices of the present invention may be made by a polymer-solvent phase separation technique, such as lyophilization. Generally, however, a polymer solution can be separated into two phases by any one of the four techniques: (a) thermally induced gelation/crystallization; (b) non-solvent induced separation of solvent and polymer phases; (c) chemically induced phase separation, and (d) thermally induced spinodal decomposition. The polymer solution is separated in a controlled manner into either two distinct phases or two bicontinuous phases. Subsequent removal of the solvent phase usually leaves a porous structure of density less than the bulk polymer and pores in the micrometer ranges. (See Microcellular Foams Via Phase Separation, J. Vac. Sci. Technolol., A. T. Young, Vol. 4(3), May/Jun 1986.)

The steps involved in the preparation of these foams include choosing the appropriate solvents for the polymers to be lyophilized and preparing a homogeneous solution of the polymer in the solution. The polymer solution then is subjected to a freezing and a vacuum drying cycle. The freezing step phase-separates the polymer solution and the vacuum drying step removes the solvent by sublimation and/or drying, thus leaving a porous polymer structure, or an interconnected open-cell porous foam.

Suitable solvents that may be used in the preparation of the foam scaffold component include, but are not limited to, formic acid, ethyl formate, acetic acid, hexafluoroisopropanol (HFIP), cyclic ethers (e.g., tetrahydrofuran (THF), dimethylene fluoride (DMF), and polydioxanone (PDO), acetone, acetates of C2 to C5 alcohols (e.g., ethyl acetate and t-butylacetate), glyme (e.g., monoglyme, ethyl glyme, diglyme, ethyl diglyme, triglyme, butyl diglyme and tetraglyme), methylethyl ketone, dipropyleneglycol methyl ether, lactones (e.g., δ-valerolactone, γ-valerolactone, β-butyrolactone, γ-butyrolactone), 1,4-dioxane, 1,3-dioxolane, 1,3-dioxolane-2-one (ethylene carbonate), dimethlycarbonate, benzene, toluene, benzyl alcohol, p-xylene, naphthalene, tetrahydrofuran, N-methyl pyrrolidone, dimethylformamide, chloroform, 1,2-dichloromethane, morpholine, dimethylsulfoxide, hexafluoroacetone sesquihydrate (HFAS), anisole and mixtures thereof. Among these solvents, a preferred solvent is 1,4-dioxane. A homogeneous solution of the polymer in the solvent is prepared using standard techniques.

The applicable polymer concentration or amount of solvent that may be utilized will vary with each system. Generally, the amount of polymer in the solution can vary from about 0.5% to about 90% by weight and, preferably, will vary from about 0.5% to about 30% by weight, depending on factors such as the solubility of the polymer in a given solvent and the final properties desired in the foam scaffold.

In one embodiment, solids may be added to the polymer-solvent system to modify the composition of the resulting foam surfaces. As the added particles settle out of solution to the bottom surface, regions will be created that will have the composition of the added solids, not the foamed polymeric material.

Alternatively, the added solids may be more concentrated in desired regions (i.e., near the top, sides, or bottom) of the resulting tissue implant, thus causing compositional changes in all such regions. For example, concentration of solids in selected locations can be accomplished by adding metallic solids to a solution placed in a mold made of a magnetic material (or vice versa).

A variety of types of solids can be added to the polymer-solvent system. Preferably, the solids are of a type that will not react with the polymer or the solvent. Generally, the added solids have an average diameter of less than about 1.0 mm and preferably will have an average diameter of about 50 to about 500 microns. Preferably the solids are present in an amount such that they will constitute from about 1 to about 50 volume percent of the total volume of the particle and polymer-solvent mixture (wherein the total volume percent equals 100 volume percent).

Exemplary solids include, but are not limited to, particles of demineralized bone, calcium phosphate particles, Bioglass particles or calcium carbonate particles for bone repair, leachable solids for pore creation and particles of bioabsorbable polymers not soluble in the solvent system that are effective as reinforcing materials or to create pores as they are absorbed, non-bioabsorbable materials, and biologically-derived bioabsorbable materials.

Suitable leachable solids include nontoxic leachable materials such as salts (e.g., sodium chloride, potassium chloride, calcium chloride, sodium tartrate, sodium citrate, and the like), biocompatible mono and disaccharides (e.g., glucose, fructose, dextrose, maltose, lactose and sucrose), polysaccharides (e.g., starch, alginate, chitosan), water soluble proteins (e.g., gelatin and agarose). The leachable materials can be removed by immersing the foam with the leachable material in a solvent in which the particle is soluble for a sufficient amount of time to allow leaching of substantially all of the particles, but which does not dissolve or detrimentally alter the foam. The preferred extraction solvent is water, most preferably distilled-deionized water. Such a process is described in U.S. Patent No. 5,514,378. Preferably the foam will be dried after the leaching process is complete at low temperature and/or vacuum to minimize hydrolysis of the foam unless accelerated absorption of the foam is desired.

Suitable non-bioabsorbable materials include biocompatible metals such as stainless steel, cobalt chrome, titanium and titanium alloys, and bioinert ceramic particles (e.g., alumina, zirconia, and calcium sulfate particles). Further, the non-bioabsorbable materials may include polymers such as polyethylene, polyvinylacetate, polymethylmethacrylate, silicone, polyethylene oxide, polyethylene glycol, polyurethanes, and natural biopolymers (e.g., cellulose particles, chitin, keratin, silk, and collagen particles), and fluorinated polymers and copolymers (e.g., polyvinylidene fluoride).

It is also possible to add solids (e.g., barium sulfate) that will render the tissue implants radio opaque. The solids that may be added also include those that will promote tissue regeneration or regrowth, as well as those that act as buffers, reinforcing materials or porosity modifiers.

Suitable biological materials include solid particles of small intestine submucosa (SIS), hyaluronic acid, collagen, alginates, chondroitin sulfate, chitosan, and blends thereof. The solids may contain the entire structure of the biological material or bioactive fragments found within the intact structure.

In addition, effectors, including bioactive molecules or cells, such as platelet rich plasma, bone marrow cells, and a combination of the two, can be added or injected into the implant at the point of care.

As noted above, porous, tissue-engineered scaffold implant devices of the present invention are made by injecting, pouring, or otherwise placing, the appropriate polymer solution into a mold set-up comprising a mold for forming the foam scaffold component about the fixation component, and the fixation component. The mold set-up is cooled in an appropriate bath or on a refrigerated shelf and then lyophilized, thereby providing a tissue-engineered scaffold implant device. In the course of forming the foam scaffold component, it is believed to be important to control the rate of freezing of the polymer-solvent system. The type of pore morphology that is developed during the freezing step is a function of factors such as the solution thermodynamics, freezing rate, temperature to which it is cooled, concentration of the solution, and whether homogeneous or heterogeneous nucleation occurs. After having the benefit of the present invention, one of ordinary skill in the art can readily optimize the parameters without undue experimentation.

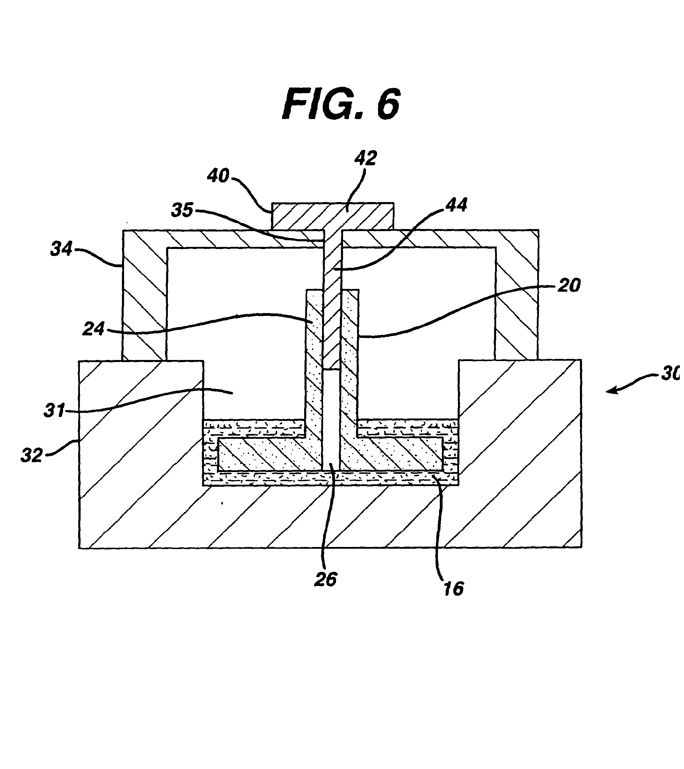

Figure 6 illustrates mold-set up 30 for use with the present invention, including mold 32 and support bracket 34. Support bracket 34 includes through-hole 35, which is aligned over well 31 of mold 32. Holder 40, including head component 42 and connector component 44, is in contact with both support bracket 34 and fixation component 20. The size of connector component 44 is such that it may pass through through-hole 35 of support bracket 34. The size of head component 42 of holder 40 is too large to pass through through-hole 35 of support bracket 34, resulting in connector component 44 being aligned over well 31 of mold 32. Fixation component 20 includes fixation component through-hole 26 matched in size to the size of connector component 44, so that there is a friction-fit between the two. By partially disposing connector component 44 in fixation component through hole 26, connector component 44 is attached to fixation component 20. The result is fixation component 20 being aligned over the well of mold 32.

Polymer-solvent solution 16 is disposed in well 31 of mold 32 at a level such that, when lyophilized, the resulting polymer foam will partially encapsulate fixation component 20, thus providing an effective means for fixedly attaching foam scaffold 12 to fixation component 20 as shown in Figures 1 to 3. The orientation of fixation component 20 within polymer-solvent solution 16 determines the orientation of fixation component 20 within polymer foam scaffold component 12 of implant 10. Therefore, care is taken in order to align fixation component 20 in solution 16 in a precise, accurate and repeatable manner, depending on the particular use anticipated for the particular device being manufactured.

Mold set-up 30 then is subjected to lyophilization as discussed herein. The result is device 10, where fixation component 20 is partially or fully imbedded in polymer foam scaffold component 12. Whether partially or fully embedded, it is necessary that the extent of encapsulation be effective to maintain fixed attachment of the foam scaffold and fixation components as discussed herein.

Although the means of aligning fixation component 20 in well 31 of mold 32 include support bracket 34 and a friction-fit between connector component 44 of holder 40 and fixation component 20, other means will be readily apparent to one skilled in the art.

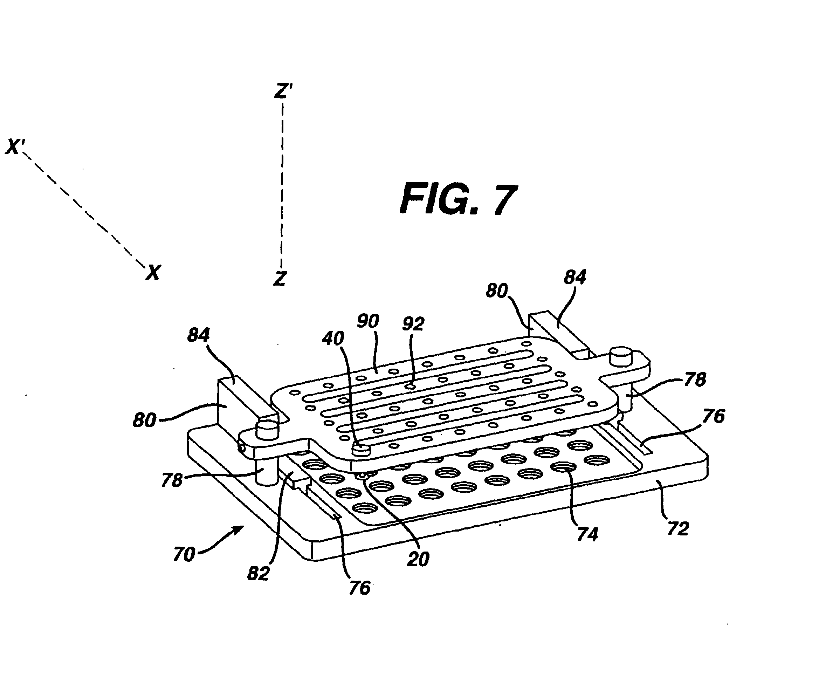

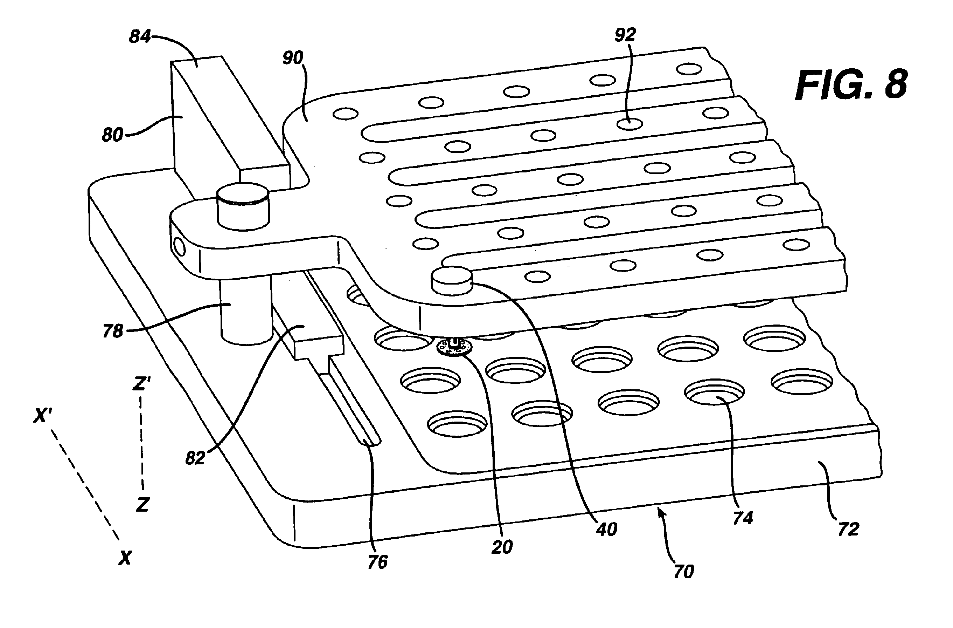

Mold 30 is able to produce one tissue-engineered scaffold implant device 10 of the present invention. A preferred embodiment for producing multiple devices 10 is shown in Figures 7 and 8. The figures show mold carrier 70, which includes base 72, plate 90, pins 78, and plate supports 80. Base 72 includes mold wells 74 and slots 76. Pins 78 may be attached to base 72 by one of several common means. For example, base 72 may have blind holes with female threads that are matched to male threads on the surfaces of pins 78. Pins 78 also may be soldered to base 72. Plate supports 80 include top steps 84 and bottom steps 82, and are disposed in slots 76 of base 72 such that plate supports 80 may translate along axis X―X'. Plate 90 includes through-holes 92 matched to the dimensions of pins 78 such that when pins 78 are disposed in plate 90 they may translate along axis Z―Z'. Plate holes 92 are located on plate 90 so as to be aligned over mold wells 74 when pins 78 are disposed in plate 90.

Figures 7 and 8 also show fixture component 20 and holder 40 disposed in plate 90 in the manner shown on Figure 6.

In manufacturing, multiple fixture/holder units may be placed in plate 90 so as to be aligned over mold wells 74. Initially, plate 90 is disposed on top steps 84 of plate supports 80. Mold wells 74 then are filled to the appropriate level with polymer solvent solution 16, as shown in Figure 6. Plate supports 80 then are translated in slots 76 along the X―X' axis so that plate 90 slides along axis Z'―Z of pins 78 and is then disposed on bottom steps 82 of plate supports 80. The movement of plate 90 results in fixation component 20 being disposed in mold wells 74 such that when polymer solution 16 is lyophilized, the resulting polymer foam 12 scaffold will partially encapsulate fixation component 20, as shown in Figures 1 to 3. One can see that plate supports 80 with bottom steps 82 of varying height can be used to control the depth that fixation component 20 will be immersed within mold wells 74 to achieve the desired height of foam scaffold component 12.

In yet another embodiment, the three-dimensional lyophilized foam scaffolds of the present invention can be covered with a sheet of a biodegradable and porous polymer sheet produced by an electrostatic spinning process. The electrostatically spun fabric can provide the scaffold with enhanced mechanical properties, the ability to hold sutures, or to act as a barrier to retard undesirable tissue growth. The electrostatically spun fabric can be made of any biocompatible material, including bioabsorbable materials such as polylactic acid (PLA), polyglycolic acid (PGA), polycaprolactone (PCL), polydioxanone (PDO), trimethylene carbonate (TMC), polyvinyl alcohol (PVA), copolymers or blends thereof. The electrostatically spun fabric also can be made from bipolymers such as hyaluronic acid, collagen, alginates, chitosan, and blends thereof.

In yet another embodiment of the present invention, the polymers and blends can be used as a therapeutic agent release matrix. To form this matrix, the polymer would be mixed with a therapeutic agent prior to forming the device. The variety of different therapeutic agents that can be used in conjunction with the polymers of the present invention is vast. In general, therapeutic agents which may be administered via the pharmaceutical compositions of the invention include, without limitation: antiinfectives, such as antibiotics and antiviral agents; chemotherapeutic agents (i.e. anticancer agents); anti-rejection agents; analgesics and analgesic combinations; anti-inflammatory agents; hormones such as steroids; analgesics; growth factors, including bone morphogenic proteins (i.e. BMP's 1-7), bone morphogenic-like proteins (i.e. GFD-5, GFD-7 and GFD-8), epidermal growth factor (EGF), fibroblast growth factor (i.e. FGF 1-9), platelet derived growth factor (PDGF), insulin like growth factor (IGF-I and IGF-II), transforming growth factors (i.e. TGF-β I-III), vascular endothelial growth factor (VEGF), or small molecules that affect the up regulation of specific growth factors; and other naturally derived or genetically engineered proteins, polysaccharides, glycoproteins, or lipoproteins. These growth factors are described in The Cellular and Molecular Basis of Bone Formation and Repair by Vicki Rosen and R. Scott Thies, published by R.G. Landes Company, hereby incorporated herein by reference.

Matrix materials for the present invention may be formulated by mixing one or more therapeutic agents with the polymer. Alternatively, a therapeutic agent could be coated on to the polymer, preferably with a pharmaceutically acceptable carrier. Any pharmaceutical carrier can be used that does not dissolve the polymer. The therapeutic agent may be present as a liquid, a finely divided solid, or any other appropriate physical form. Typically, but optionally, the matrix will include one or more additives, such as diluents, carriers, excipients, stabilizers or the like.

The amount of therapeutic agent will depend on the particular drug being employed and medical condition being treated. Typically, the amount of drug represents about 0.001 percent to about 70 percent, more typically about 0.001 percent to about 50 percent, most typically about 0.001 percent to about 20 percent by weight of the matrix. The quantity and type of polymer incorporated into the drug delivery matrix will vary depending on the release profile desired and the amount of drug employed.

Upon contact with body fluids, the polymer undergoes gradual degradation (mainly through hydrolysis) with concomitant release of the dispersed drug for a sustained or extended period. This can result in prolonged delivery (over, say 1 to 5,000 hours, preferably 2 to 800 hours) of effective amounts (say, 0.0001 mg/kg/hour to 10 mg/kg/hour) of the drug. This dosage form can be administered as is necessary depending on the subject being treated, the severity of the affliction, the judgment of the prescribing physician, and the like. Following this or similar procedures, those skilled in the art will be able to prepare a variety of formulations.

In another embodiment, the fixation device can be fabricated from biocompatible ceramics such as hydroxyapatite, tricalcium phosphate, or blends with biocompatible and resorbable synthetic, or metal alloys. The devices could also be made from natural materials (ie allograft bone, xenograft bone, coral etc.)

In another alternative embodiment of the invention, the lyophilized foam may be modified either through physical or chemical means to contain biological or synthetic factors that promote attachment, proliferation, differentiation, and matrix synthesis of targeted cell types. Furthermore, the bioactive factors may also comprise part of the matrix for controlled release of the factor to elicit a desired biological function. Another embodiment would include delivery of small molecules that affect the up regulation of endogenous growth factors. Growth factors, extracellular matrix proteins, and biologically relevant peptide fragments that can be used with the matrices of the current invention include, but are not limited to, members of TGF-□ family, including TGF-□1, 2, and 3, bone morphogenic proteins (BMP-2, -12, and -13), fibroblast growth factors-1 and -2, platelet-derived growth factor-AA, and -BB, platelet rich plasma, vascular endothelial cell-derived growth factor (VEGF), pleiotrophin, endothelin, tenascin-C, fibronectin, vitronectin, tropoelastin, and biological peptides containing cell- and heparin-binding domains of adhesive extracellular matrix proteins such as fibronectin and vitronectin. The biological factors may be obtained either through a commercial source or isolated and purified from a tissue.

In yet another alternative embodiment, the lyophilized structures of the present invention can be seeded or cultured with appropriate cell types prior to implantation for the targeted tissue. Cells which can be seeded or cultured on the matrices of the current invention include, but are not limited to, bone marrow cells, mesenchymal stem cells, stromal cells, stem cells, embryonic stem cells, chondrocytes, osteoblasts, precursor cells derived from adipose tissue, and genetically transformed cells that can release the desired growth factor, extracellular matrix protein, signaling molecule or provide an immunoprotective site for allogeneic or xenogeneic cells. The cells can be seeded on the scaffolds of the present invention for a short period of time (< 1 day) just prior to implantation, or cultured for longer (> 1 day) period to allow for cell proliferation and matrix synthesis within the seeded scaffold prior to implantation.

The following examples are illustrative of the principles and practice of the invention, although not limiting the scope of the invention. Numerous additional embodiments within the scope and spirit of the invention will become apparent to those skilled in the art.

In the examples, the polymers and monomers were characterized for chemical composition and purity (NMR, FTIR), thermal analysis (DSC), and molecular weight by conventional analytical techniques.

Inherent viscosities (I.V., dL/g) of the polymers and copolymers were measured using a 50 bore Cannon-Ubbelhode dilution viscometer immersed in a thermostatically controlled water bath at 25°C utilizing chloroform or hexafluoroisopropanol (HFIP) as the solvent at a concentration of 0.1 g/dL.

In these examples certain abbreviations are used, such as PCL to indicate polymerized ε-caprolactone, PGA to indicate polymerized glycolide, PLA to indicate polymerized (L)lactide. Additionally, the percentages in front of the copolymer indicates the respective mole percentages of each constituent.

### Example 1:

This example describes the process for making a tissue-engineered scaffold implant device of the present invention.

Bioabsorbable fixation components were manufactured using an injection molding process. The design of the fixation component used is the same as that depicted in Figure 3a. The polymer used to manufacture the fixation components was a copolymer of 85% PLA and 15% PGA (85/15 PLA/PGA) produced by Purac (Gorinchem, The Netherlands), with an I.V. of 1.79 dL/g as measured in chloroform. The injection molder (Niigata NN35MI) had a barrel diameter of 18 mm. The hopper was fitted with a nitrogen purge to keep the polymer dry. The feed, transition and compression zone temperatures were 185°C, 185°C and 191°C, respectively. The die and mold temperatures were 191°C and 24°C, respectively. The maximum injection speed was 80 mm/s and maximum injection pressure was 85 Kgf/cm². The hold pressure was 70 Kgf/cm². The total time for injection and hold was 3 seconds and the cooling time at the end of hold cycle was 20 seconds.

A solution of the polymer to be lyophilized into a foam scaffold then was prepared. The polymer used to manufacture the foam components was a copolymer of 35% PCL and 65% PGA (35/65 PCL/PGA) produced by Birmingham Polymers Inc. (Birmingham, AL) with an I.V. of 1.79 dL/g, as measured in HFIP at 30°C. A 90/10 weight ratio of 1,4-dioxane/(35/65 PCL/PGA) was weighed out. The polymer and solvent then were placed into a flask, which in turn was put into a water bath and stirred at 70°C for 5 hrs. The solution then was filtered using an extraction thimble (extra coarse porosity, type ASTM 170-220 (EC)) and stored in a flask.

The solution was added to the mold such that the solution reached a level of 2.0 mm in the mold. Several fixation components were suspended approximately 1.0 - 1.5 millimeters above the bottom surface of a mold as depicted in Figure 6. Thus, the bottom face of the scaffold support component was below the level of the solution. This mold suspended the fixation components in the polymer-solvent solution in a precise, accurate, and repeatable manner.

The mold assembly then was placed on the shelf of the lyophilizer and the freeze-dry sequence begun. A laboratory scale lyophilizer (Model Duradry, from FTS Kinetics, Stone Ridge, NY), was used in this example. The freeze dry sequence used in this example was: 1) 20°C for 15 minutes; 2) -5°C for 120 minutes; 3) -5°C for 90 minutes under vacuum 100 mT; 4) 5°C for 90 minutes under vacuum 100 mT; 5) 20°C for 90 minutes under vacuum 100 mT.

After the cycle was completed, the mold assembly was taken out of the freeze drier and allowed to degas in a vacuum hood for 2 to 3 hours. The devices then were stored under nitrogen.

In the resulting devices, the fixation components were fixedly attached to the polymer foam scaffold components. Figures 4 and 5 are scanning electron micrographs (SEMs) of the cross-section of the rigid fixation component embedded in the polymer foam scaffold. The SEM clearly shows the lyophilized foam scaffold surrounding and encapsulating the rigid fixation component.

### Example 2:

This example illustrates that the devices disclosed in this invention allow for in vitro invasion of chondrocytes.

Primary chondrocytes were isolated from bovine shoulders as described by Buschmann, et al., in *J. Orthop. Res.,* 10, 745, (1992). Bovine chondrocytes were cultured in Dulbecco's modified eagles medium (DMEM-high glucose) supplemented with 10% fetal calf serum (FCS), 10 mM HEPES, 0.1 mM nonessential amino acids, 20 µg/ml L-proline, 50 µg/ml ascorbic acid, 100 U/ml penicillin, 100 µg/ml streptomycin and 0.25 µg/ml amphotericin B (growth media). Half of the medium was replenished every other day.

Tissue engineered scaffold implant devices were prepared as described in Example 1. The devices were sterilized for 20 minutes in 70% ethanol followed by five rinses of phosphate-buffered saline (PBS).

Freshly isolated bovine chondrocytes were seeded at 5 x 10⁶ cells (in 50 µl medium) by a static seeding method in hydrogel-coated plates (ultra low cluster dishes, Costar). Following 6 hours of incubation in a humidified incubator, the devices were replenished with 2 ml of growth media. The devices were cultured statically for up to 6 weeks in growth media.

Devices harvested at 6 weeks were fixed in 10% buffered formalin, embedded in paraffin and sectioned. Sections were stained with Safranin-O (SO; sulfated glycosaminoglycans-GAG's). Three samples were sectioned and stained.

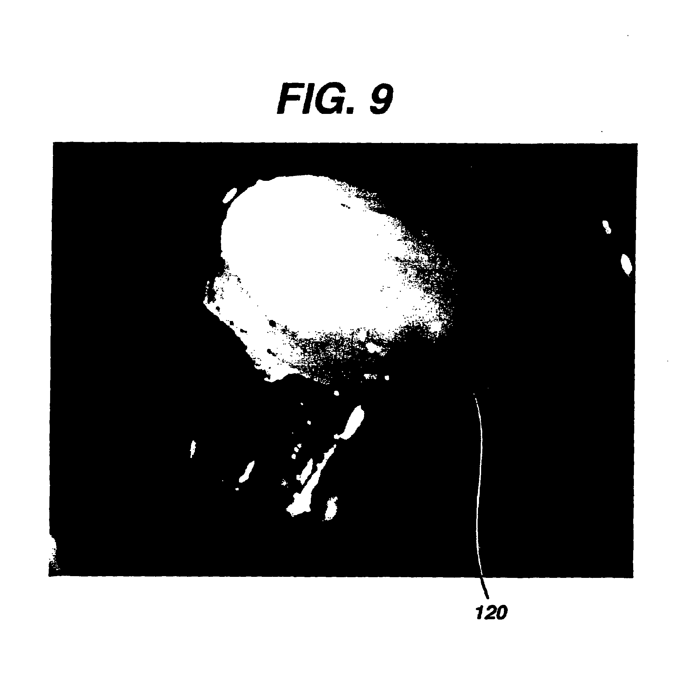

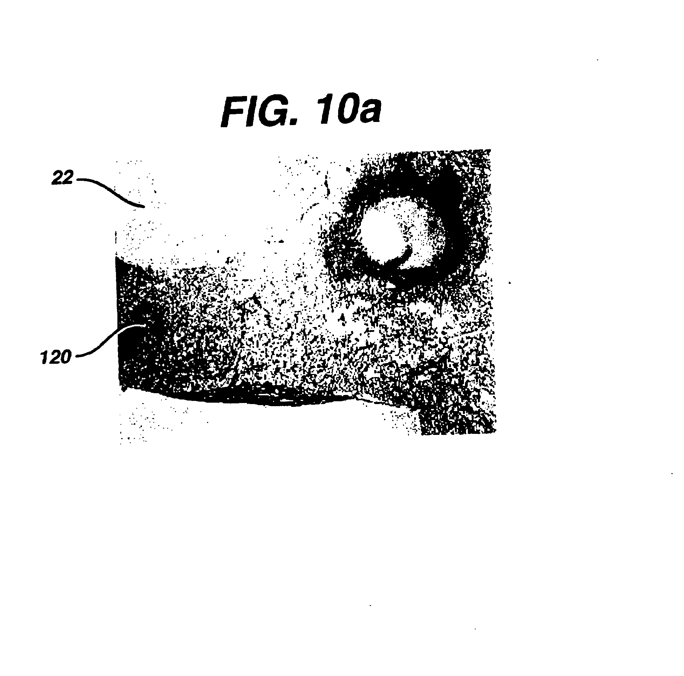

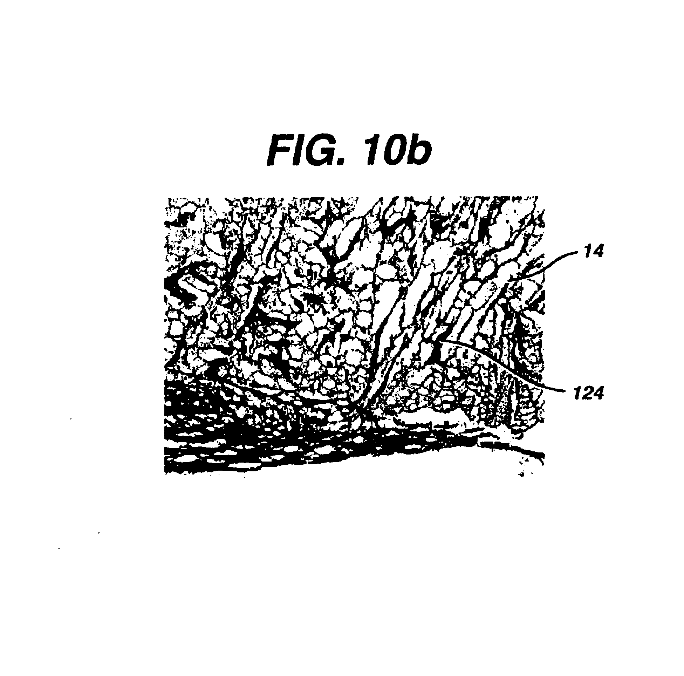

Figure 9 is a photographic image of a device that was cultured with cells for 6 weeks. As shown therein, cell-infiltrated foam scaffold 120 encapsulates scaffold support 22 (not shown). Figures 10a and 10b are histological sections of the device. Figure 10a shows scaffold support 22 of the fixation component and cell-infiltrated foam scaffold 120. Figure 10b is a magnified portion of Figure 10a, showing stained cells 124 and pores 14 of the foam scaffold component.

In summary, the architecture of the foam scaffold supported cell migration into the affixed scaffold, with the majority of cell invasion through the bottom of the scaffold.

### Example 3:

This example describes the process for making of a tissue-engineered scaffold implant device of the present invention where the foam component is of an alternate composition.

Bioabsorbable fixation components were manufactured using an injection molding process as described in Example 1.

A solution of the polymer to be lyophilized into a foam then was prepared. The polymer used to manufacture the foam components was a 50/50 mix of 60% PLA and 40% PCL (60/40 PLA/PCL) copolymer and 35% PCL and 65% PGA (35/65 PCL/PGA) copolymer. The 60/40 PLA/PCL was produced by Ethicon, Inc. (Somerville, NJ) and had an I.V. of 1.45 dL/g as measured in HFIP at 25°C. The 35/65 PCL/PGA was produced by Birmingham Polymers Inc. (Birmingham, AL) and had an I.V. of 1.79 dL/g as measured in HFIP at 30 °C. A 90/10 weight ratio of 1,4-dioxane/(50/50 copolymer mix) was weighed out. Next, the polymer and solvent were placed into a flask, which in turn was put into a water bath and stirred at 70°C for 5 hrs. Afterwards, the solution was filtered using an extraction thimble (extra coarse porosity, type ASTM 170-220 (EC)) and stored in a flask.

The set-up of the mold assembly, as well as the lyophilization process used to form the devices was the same as that of Example 1.

### Example 4:

This example describes the *in vivo* implantation of the device described in Example 3 in an osteochondral defect model. The objective of this study was to evaluate short-term integrity of the fixation component and lyophilized foam scaffold.

Tissue engineered scaffold implant devices were prepared as described in Example 3. The devices were sterilized in ethylene oxide (EtO) gas.

Animals were housed and cared for at Ethicon, Inc. (Somerville, NJ) under an approved institutional protocol. Three neutered male adult Neubian goats (50-65 Kg) were used in the study. An analgesic, buprenorphine hydrochloride, was administered subcutaneously (0.005 mg/kg) about 2-3 hrs before the start of the surgery. Anesthesia was induced in each goat with an intravenous bolus of Ketamine at 11.0 mg/kg and Diazepam at 0.5 mg/kg both given simultaneously intravenously. Next, animals were intubated and maintained in a plane of deep anesthesia with 3% Isoflurane and an oxygen flow rate of 11-15 ml/kg/min. A gastric tube was placed to prevent bloating. Cefazolin sodium (20 mg/kg) was administered intravenously preoperatively.

A lateral para-patellar incision was made and the patella dislocated medially in the right leg. With the knee fully flexed, a 7-mm diameter hole was made approximately 0.5 cm lateral from the condylar medial/lateral center and approximately 1.3 cm posterior to the anterior edge of the condyle cartilage edge. Another 7 mm defect was also created in the proximal-distal center of the lateral face of the trochlear groove to a depth of 2 mm. After drilling the defects, each joint was flushed with saline and the fixation device with the lyophilized scaffold was press fit into the 7 mm defect site. The joint capsule was closed with a polypropylene monofilament suture, sold under the tradename PROLENE (by Ethicon, Inc., Somerville, NJ) or a suture sold under the tradename VICRYL (Ethicon, Inc., Somerville, NJ), using simple continuous suture pattern. Following the surgery, the goats were placed in a Schroeder-Thomas splint with an aluminum frame for 2 weeks to allow for partial weight bearing of the right stifle.

The animals were sacrificed after eight weeks, and the defect sites (medial condyle and trochlear groove) were remove, trimmed, and fixed in 10 percent neutral buffered Formalin (20X the tissue volume).

The samples were trimmed, and placed in a decalcifying solution (Decalcifer I, Surgipath) containing 10/8/1/81 (weight percent) formaldehyde/formic acid/methanol/deionized water for 2 weeks. The decalcifying solution was replaced every day for 2 weeks followed by a final rinse in tap water. The samples were, cut into 5 µm sections, and stained with Hematoxylin Eosin (H & E).

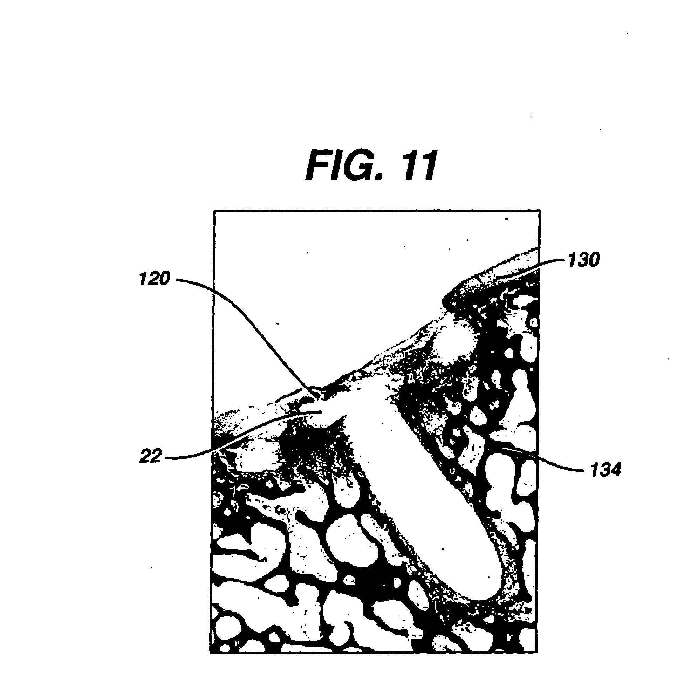

At necropsy, all of the fixation devices remained intact. Figure 11 is a typical section of explanted devices from the trochlear groove sites. The figure shows scaffold support 22 of the fixation component and cell-infiltrated foam 120, as well as native cartilage 130 and native bone 134. The figure shows evidence of tissue ingrowth at the interface between the foam component of the device and native cartilage 130.

### Example 5:

This example describes the attachment of a bioabsorbable fixation component to a bioabsorbable lyophilized foam scaffold component prepared from a biopolymer, e.g. hyaluronic acid.

Bioabsorbable rigid fixation components were manufactured using an injection molding process as described in Example 1.

A solution of hyaluronic acid (obtained from LifeCore, MN) in 0.05 M acetic acid (0.5% wt/wt) was prepared. The freeze dry sequence used in this example was: 1) -17°C for 60 minutes; 2) -5°C for 60 minutes under vacuum 100 mT; 4) 5°C for 60 minutes under vacuum 20 mT; 5) 20°C for 60 minutes under vacuum 20 mT. After the freeze-dry cycle was completed, the mold assembly was taken out of the lyophilizer and allowed to degas in a vacuum hood for 2 to 3 hours. The devices were then stored under nitrogen.

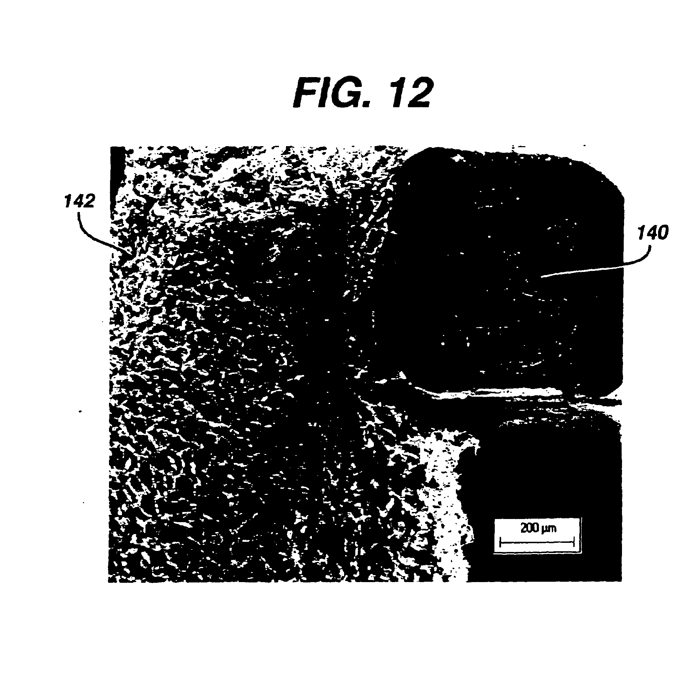

In the resulting devices, the fixation component was fixedly attached to the biopolymer foam scaffold component. As shown in Figure 12, foam scaffold component 142 surrounds and encapsulates fixation component 140. Fixation component 140 thus is embedded in scaffold component 142, thereby providing fixed attachment of one to the other.

### Example 6:

This example describes the attachment of a bioabsorbable fixation component to a bioabsorbable lyophilized foam containing a bioabsorbable nonwoven fabric, or mat, disposed therein as a reinforcing component.

Bioabsorbable rigid fixation components were manufactured using an injection molding process as described in Example 1. A solution of a 60% PLA and 40% PCL (60/40 PLA/PCL) copolymer in a suitable solvent was prepared. The 60/40 PLA/PCL was produced by Ethicon, Inc. (Somerville, NJ) and had an I.V. of 1.45 dL/g as measured in HFIP at 25°C. A 95/5 weight ratio of 1,4-dioxane/(50/50) copolymer mix was weighed out. Next, the polymer and solvent were placed into a flask, which in turn was put into a water bath and stirred at 70°C for 5 hrs. Afterwards, the solution was filtered using an extraction thimble (extra coarse porosity, type ASTM 170-220 (EC)) and stored in a flask.

The set-up of the mold assembly, as well as the lyophilization process used to form the devices was the same as that of Example 1 with the following modification. Prior to pouring the polymer solution in the mold, a nonwoven mat was placed into the mold and the fixation component was brought into contact with the nonwoven mat. This assembly, including the mold, polymer solution, fixation component, and the nonwoven fabric, was lyophilized using the cycle described in Example 1.

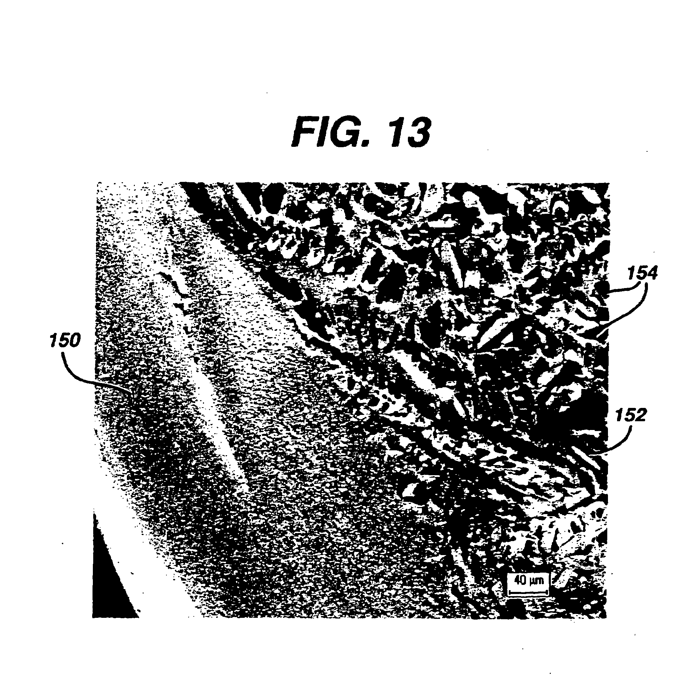

In the resulting devices, the fixation component was fixedly attached to the foam scaffold having the nonwoven mat reinforcing component disposed therein. As shown in Figure 13, fixation component 150 is embedded in foam scaffold 152. Fiber 154 of the nonwoven mat is shown embedded in foam scaffold 152. The SEM images clearly show foam scaffold 152 surrounding the nonwoven mat and encapsulating fixation component 150, thereby providing fixed attachment of the scaffold component to the fixation component.

### Example 7:

This example describes the attachment of a bioabsorbable fixation component to a lyophilized foam scaffold component prepared from an absorbable biopolymer containing a bioabsorbable nonwoven mat reinforcing component.

Bioabsorbable rigid fixation components were manufactured using an injection molding process as described in Example 1. A solution of the biopolymer to be lyophilized into a foam scaffold was then prepared. In this example, a 0.5% weight ratio of hyaluronc acid (obtained from LifeCore, MN) in 0.05 M acetic acid was prepared.

Prior to pouring the biopolymer solution in the mold, a nonwoven mat was placed into the mold and the fixation component was brought into contact with the nonwoven mat. This assembly, including the mold, biopolymer solution, fixation device, and the nonwoven fabric mat, was lyophilized using the cycle described in Example 5.

After the cycle was completed, the mold assembly was taken out of the freeze drier and allowed to degas in a vacuum hood for 2 to 3 hours. The devices were then stored under nitrogen. In the resulting devices, the fixation component was fixedly attached to the nonwoven mat encapsulated in a biopolymer foam scaffold component.

## Claims

1. A tissue scaffold implant device, comprising:
a foam tissue scaffold component having a pore structure effective to facilitate tissue infiltration and growth into the foam tissue scaffold; and
a fixation component,
wherein the foam tissue scaffold component is fixedly attached to the scaffold fixation component via partial encapsulation of the fixation component by the foam tissue scaffold component.

2. The device of claim 1 wherein the fixation component comprises tissue scaffold support means and anchor means and the foam tissue scaffold component substantially encapsulates the tissue scaffold support means.

3. The device of claim 1 or claim 2 wherein the foam scaffold component comprises a lyophilized polymer.

4. The device of claim 3 wherein the lyophilized polymer is bioabsorbable.

5. The device of any one of claims 1 to 4 wherein the fixation component comprises a bioabsorbable polymer.

6. The device of any one of claims 1 to 4 wherein the fixation component comprises a non-bioabsorbable polymer.

7. The device of claim 5 wherein the bioabsorbable polymer is selected from the group consisting of aliphatic polyesters, poly(amino acids), copoly(ether-esters), polyalkylene oxalates, polyamides, tyrosine-derived polycarbonates, poly(iminocarbonates), polyorthoesters, polyoxaesters, polyamidoesters, polyoxaesters containing amine groups, poly(anhydrides), polyphosphazenes and biopolymers.

8. The device of claim 7 wherein the aliphatic polyesters are selected from the group consisting of homopolymers and copolymers of lactide, glycolide, ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one, 1,5-dioxepan-2-one, 6,6-dimethyl-1,4-dioxan-2-one, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione and 6,8-dioxabicycloctane-7-one.

9. The device of claim 8 wherein the aliphatic polyesters are elastomeric.

10. The device of claim 7 wherein the biopolymers are selected from the group consisting of hyaluronic acid, collagen, recombinant collagen, cellulose, elastin, alginates, chondroitin sulfate, chitosan, chitin, keratin and silk.

11. The device of any one of claims 1 to 10 wherein the pore structure is open-cell.

12. The device of any one of claims 1 to 11 wherein the pores have an average diameter of from 10 to 1,000 microns.

13. The device of claim 2 wherein the scaffold support means comprises through-holes.

14. The device of any one of claims 1 to 13 further comprising a reinforcing component.

15. A method of making a tissue scaffold implant device, comprising:
placing a fixation component within a mold of selected configuration, in a selected position and orientation,
adding to the mold containing the fixation component a polymer solution comprising a selected polymeric material dissolved in a suitable solvent therefore,
separating the polymer solution in the mold into a solvent phase and a polymer phase; and
removing the solvent phase from the mold, thereby providing a foam tissue scaffold component that at least partially encapsulates the fixation component, thereby providing fixed attachment of the foam tissue scaffold component to the fixation component.

16. The method of claim 15 wherein the fixation component comprises scaffold support means and anchor means, the scaffold support means is positioned within the mold, the polymer solution substantially encapsulates the scaffold support means, and wherein upon phase separation and solvent removal, the foam tissue scaffold component produced thereby substantially encapsulates the scaffold support means, thereby providing the fixed attachment of the foam tissue scaffold component to the fixation component.

17. The method of claim 15 wherein the phase separation and solvent removal are accomplished by lyophilization.

18. The method of claim 15 further comprising placing a reinforcing component into the mold prior to placing the fixation component into the mold, such that the fixation component is in contact with the reinforcing component.

19. The process of any one of claims 15 to 18 wherein the components of the device are as defined in any one of claims 1 to 14.
